# EUROPEAN PATENT SPECIFICATION

(11) **EP 0 743 854 B1**
(45) Date of publication and mention of the grant of the patent: **27.06.2001**
(21) Application number: 94904944.9
(22) Date of filing: 07.02.1994
(51) Int. Cl.: A61K 31/28, A61K 47/12

(54) **ANTI-TUMOUR MEDICAL PREPARATION ON THE BASIS OF CARBOPLATIN AND THE METHOD OF ITS PRODUCTION**
ANTITUMORMITTEL AUF BASIS VON CARBOPLATIN UND HERSTELLUNG DESSELBEN
COMPOSITION PHARMACEUTIQUE ANTITUMORALE A BASE DE CARBOPLATINE, ET SON PROCEDE DE PRODUCTION

(43) Date of publication of application: 27.11.1996
(73) Proprietor: PLIVA, pharmaceutical industry incorporated, 10000 Zagreb (HR)
(72) Inventor: KISS, Frantisek, 619 00 Brno (CZ); KISSOVA, Ludmila, 619 00 Brno (CZ)
(74) Representative: Gleiss & Grosse
(86) International application number: CZ9400007
(87) International publication number: WO9520956

(56) References cited:
- EP-A- 0 275 559
- EP-A- 0 334 551
- EP-A- 0 401 896
- US-A- 4 140 707
- CHEMICAL ABSTRACTS, vol. 117, no. 10, 7 September 1992, Columbus, Ohio, US; abstract no. 97337, cited in the application & CS,A,270 951 (F.KISS ET AL.) 11 July 1991
- DATABASE WPI Week 9043, Derwent Publications Ltd., London, GB; AN 90-326085 (43) & JP,A,02 235 813 (FUJIMOTO J.) 18 September 1990
- CHEMICAL ABSTRACTS, vol. 113, no. 11, 10 September 1990, Columbus, Ohio, US; abstract no. 91008, & CHEM.-BIOL.INTERACT., vol.73, no.2-3, 1990 pages 337 - 351 W.E.HILL ET AL. 'SYNTHESIS,CHARACTERIZATION AND ANTI-TUMOR TESTING OF SOME PLATINUM (II) AMINE COMPLEXES CONTAINING 1,1- AND 1,2-CYCLOBUTANEDICARBOXYLATE LIGANDS'
- CHEMICAL ABSTRACTS, vol. 106, no. 16, 20 April 1987, Columbus, Ohio, US; abstract no. 125773, cited in the application & AM.J.HOSP.PHARM., vol.44, no.1, 1987 pages 124 - 130 Y.W.CHEUNG ET AL. 'STABILITY OF CISPLATIN,IPROPLATIN,CARBOPLATIN,AND TETRAPLATIN IN COMMONLY USED INTRAVENOUS SOLUTIONS'

## Description

The invention relates to an anti-tumour medical preparation whose active agent is the platinum complex cis-diammine-1,1-cyclobutanedicarboxylate platinum (II) of the formula I having the international free name carboplatin 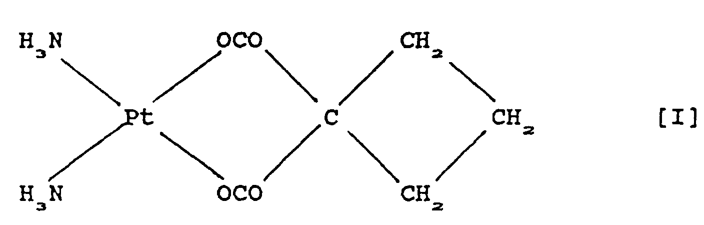 and a method for its production.

The complex I has been prepared for the first time in 1972 (US patent 4 140 707) and the problems of this generally described preparation have been disclosed in EP 0275 559.

This platinum cytostatic of the so called 2nd generation has been introduced into clinical practice in 1986 in a form of a lyophylized injection comprising mannitol as a pharmacologically acceptable trituration agent (Paraplatin - packing ticket for the preparation of the Bristol Myers, U.S.A.). In 1988, the medical preparation on the basis of carboplatin (Lachema Corp., Czech Republic) using sodium citrate as an auxiliary substance the advantage of which was disclosed in the A.O. CS 270 951 has been registered and approved for distribution.

Due to considerable consumption increase of both lyophylized compositions it became necessary to eliminate some shortcomings in the area of their production and administration.

The production of dry injection by means of lyophylization is both time and energy consuming process, nevertheless the products exhibit usually high stability. On the other hand the disadvantage is both the exacting solution preparation for infusion and the risk of carboplatin bonding to DNA leading to chromosomal aberrations. Above all those facts contributed decidedly to a decision to develop the liquid form of that cytostatic which due to handle reduction would consequently lead to minimizing the contamination of both the environment and medical personnel.

In respect of the general requirement of sufficient stability of mass-produced medical preparations it is necessary to advice on the problem complexity; it is well known that the solution of carboplatin prepared by means of dissolving the above mentioned lyophylized injection in water and other aqueous solutions (sodium chloride, glucose, dextrose, etc.), can be thought as stable and therefore therapeutically useful for only few hours, at most few days under lower temperature (Cheung, Y.W., Gradock, J.C., Vishnuvajjala, B.R., et all., Amer. J. Hosp. Pharm., 44, pp. 124-130, 1987).

The results of earlier works gained by measuring the conductivity of aqueous carboplatin solutions, affirming that it is a matter of kinetically inert complex (Cleare, M.J., Hydes, P.C., et all., Biochemie, 60, pp. 835-850, 1978), cannot be thought as conclusive.

The inventors of this invention have been engaged in a study of substitution reactions of the complex I using several high sensitive both separation and identification methods of intermediates in the system of running processes.

The conclusion of the study can be summarized in the following points:
1. Aqueous solution of carboplatin under absence of other compounds undergoes above all a slow hydrolysis, i.e., the substitution of the 1,1-cyclobutanedicarboxylic ligands by aqueous ligand occurs, while when using the high performance liquid chromatography (HPLC), the formation of toxic cis-diamminedi-aquaplatinum (II) complex was recorded. During longer storage, especially at elevated temperature, the elimination of NH₃ ligands is possible.
2. Addition of other compounds into solution (inorganic or organic acids and their salts, etc.), does not lead to higher carboplatin stability. On the contrary, it leads rather to a variety of less soluble and more toxic complexes, for instance cis-diammineoxalate platinum (II), what has been disclosed in the very generally defined claims of EP Application 0401896. An exception in this respect is above all the 1,1-cyclobutanedicarboxylic acid and its salts.
3. Addition of most organic compounds, sometimes already in trace amounts, especially at elevated temperature, causes the carboplatin reduction to colloid platinum. In solutions under atmosphere containing no oxygen also an autocatalytic carboplatin decomposition can occur.
4. It must be admitted that the addition of oxidative compounds into carboplatin solution inhibits its reduction, however it leads to formation of a tetravalent platinum complex analogue. The oxygen behaves as a very mild oxidative agent in this respect, and his presence in small amounts is preferable.
5. The extent and the rate of the "decomposition" processes observed depends expressly not only on the temperature, but also on the concentration of the added compound, pH value of the solution and the effect of a short-wave light.

The result of the above-mentioned study is a new anti-tumour medical preparation on the basis of carboplatin, characterized in that the stabilized sterile aqueous solution has a pH value in the range from 2.5 to 7.0 and contains 1,1-cyclobutanedicarboxylic acid and/or its sodium and/or potassium and/or ammonium salts in an amount of from 5·10⁻⁴mg/ml to 2·10⁻¹mg/ml, at least 1·10⁻⁴mg/ml of which is present in the form of mono- and/or bi-valent anion.

In spite of acceptable carboplatin stability in a relatively large range of pH values, the best results were obtained with pH between 4.0 and 6.5, the best long term stability being with pH in the range from 4.5 to 6.0.

Comparing the aqueous carboplatin solution containing no ingredients the positive influence of the 1,1-cyclobutanedicarboxylic acid and/or its sodium and/or potassium and/or ammonium salts is evident and already at very low concentrations suppresses the aquacomplex formation. Very good stability in the range from 1.10⁻³ mg/ml to 2·10⁻¹ mg/ml was reached, an optimum stability in the range from 5·10⁻³ mg/ml to 1·10⁻¹ mg/ml, however at least 1·10⁻⁴ mg/ml of this acidoligand should be present in the form of mono- and/or bi-valent anion.

The presence of an ammonium ligand (NH₃) source in solution in a form of ammonia and/or its protonized form in concentration from 1·10⁻⁵ mg/ml to 6·10⁻² mg/ml is desirable especially for the stabilization of carboplatin stored under uncontrolled conditions, i.e. at possible elevated temperature, when the possibility of NH₃ ligands elimination from carboplatin complex increases. In addition to this the use of aqueous ammonia solution for easy adjusting of pH is advantageous as well.

Anti-tumour preparation on the carboplatin basis can comprise beside above mentioned compounds from the pharmacological point of view other acceptable ingredients, for instance from the group of polysaccharides. Advantageously, for instance α-cyclodextrin in an amount of from 0.1 mg/ml to 10 mg/ml can be used and synergistic effect in both the stability and carboplatin solubility can be reached.

Preparation on the basis of above mentioned carboplatin composition from the clinical administration point of view can be produced advantageously in a concentration of from 0.1 to 25 mg per 1 ml solution. The preparation can be thus administered directly, for instance in a form of continuous administration or after dilution with a suitable infusion solution, for instance 5% glucose (dextrose).

The preparation of the above mentioned composition can be produced by means of adding ingredients under aseptic conditions successively or simultaneously under agitation into water for injection purpose, dissolved, the pH value of the solution is adjusted, if necessary, sterilized preferably by means of ultrafiltration and then under aseptic conditions filled into containers determined for injection and/or infusion preparation while during preparation, filling into containers and storage the solution is kept under inert or oxidative atmosphere and under exclusion of the short-wave light. for the

The advantages of the composition and the method for the production of the new carboplatin medical form according to this invention from the stability point of view follows among others from the data on active substance concentration in solution with different auxiliary compound composition after 6 months storage at room temperature.
Initial state: carboplatin concentration in all solutions
cₒ = 10.0 mg/ml = 100%, pH = 5.0 ± 0.1

| State after 6 months storage: | | |
|---|---|---|
| | Solutions of auxiliary compounds | carboplatin content |
| 1a | 0.02% ammonium 1,1-cyclobutanedicarboxylate | 100% |
| 1b | 0.1% potassium 1,1-cyclobutanedicarboxylate | 100% |
| 1c | 0.5% 1,1-cyclobutanedicarboxylic acid | 98% |
| 1d | 0.01%(NH₄Na) 1,1-cyclobutanedicarboxylate | 100% |
| 1e | 0.001%(NH₄Na) 1,1-cyclobutanedicarboxylate | 100% |
| 2 | 0.1% ammonium phosphate | 95% |
| 3 | 0.1% ammonium citrate | 90% |

Stability of the carboplatin in a solution according to this invention is conclusively better than in the comparative solutions containing phosphate or citrate buffers.

The advantage of the anti-tumour preparation according to this invention is a practically identical biologic activity compared with that of the lyophylized preparation introduced into routine therapy earlier. The pharmacodynamic effect (acute toxicity and the anti-tumour effect) does not differ statistically significantly from the fresh-made solution of lyophylized injection. The biologic harmlessness of 1,1-cyclobutanedicarboxylic acid and its salts is advantageous and is demonstrated by the LD₅₀ value for mice which is much higher than 1000 mg/kg of body weight, while when using the new preparation on the carboplatin basis only 0.3 mg of "free" acid and/or their salts per 1 kg of the body weight is introduced to the man. The administration security of the new preparation on the carboplatin basis in infusion form after dilution with 5% glucose in volume ratio 1:10 has been demonstrated during clinical tests of the preparations with 50, 150, 450 and 600 mg respectively, of the active substance.

### Examples

### Example 1 (Comparative example)

22.5 g of 1,1-cyclobutanedicarboxylic acid were dissolved under aseptic condition in 88.00 kg of fresh redestilled apyrogenic water having a temperature of 25°C and by adding a 2.5% solution of ammonia the pH 5.5 was adjusted. Subsequently 900 g of carboplatin under agitation and short-wave light exclusion was added. When the substance was completely dissolved, redestilled water up to total weight of 90.00 kg was added thereto, the solution was saturated by ultrafiltered nitrogen, sterilized by means of ultrafiltration and subsequently under contindus nitrogen purge filled in doses of 5.3 ml into vials, closed up by rubber stopper and sealed with an aluminium cap.

### Example 2

Anti-tumour preparation according to Example 1 comprising 150 mg of carboplatin per vial. (Comparative example). 30 g of ammonium 1,1-cyclobutanedicarboxylate were dissolved under agitation and purge by nitrogen containing approximately 0.5% of oxygen, in 88.00 kg of fresh redestilled apyrogenic water at a temperature of 22°C. 900 g of carboplatin was dissolved under exclusion of short-wave light in the above mentioned solution having pH value 5.3 and finally 1.07 kg of water was added thereto. The solution was sterilized similarly to Example 1 and filled into vials in doses of 15.5 ml.

### Example 3

Anti-tumour preparation comprising 10 mg of carboplatin and both 0.02 mg sodium and ammonium 1,1-cyclobutanedicarboxylate in 1 ml of solution having pH value 5.8.
The process was made according to Example 2 and the sterile solution was filled in doses of 61.5 ml. 1450 vials each containing at least 600 mg of active substace were obtained this way.

### Example 4

Anti-tumour preparation comprising 450 mg of carboplatin per vial.
152.0 g of carboplatin synthetized in a special way to contain about 1 g of ammonium 1,1-cyclobutanedicarboxylate were dissolved at a temperature of 25°C and under aseptic conditions in 14.00 kg of water for injection purpose. To the solution thus obtained, having the pH 5.7, water for injection purpose up to total weight of 15.00 kg was added, the solution was saturated by nitrogen, sterilized by means of ultrafiltration and filled in doses of 46.0 ml into vials containing at least 450 mg of carboplatin each.

During 24 months storage of those injections at a temperature below + 8°C or 20°C respectively, at least 99,5% of active substance - carboplatin - was conserved. Because the concentration of more toxic cisplatin has not increased during this time period as well (was lower than 0.1%) the chromatographic purity of the preparation was also acceptable. The total concentration of other related platinum complexes increased comparing with the initial stage only from 0.05 to 0.2 or 0.35% respectively, so that the solution can be considered as very stable.

### Example 5

Anti-tumour preparation comprising 15 mg of carboplatin, 0.1 mg potassium 1,1-cyclobutanedicarboxylate and 5 mg of α-cyclodextrin in 1 ml of solution having pH 4.8.
7.5 g of α-cyclodextrin, 0.15 g of potassium 1,1-cyclobutanedicarboxylate and 22.5 g of carboplatin were dissolved at a temperature of 25 - 28°C in 1.40 kg of water for injection purpose. To the solution having pH 4.8 water for injection purpose was added up to total weight of 1.50 kg, the solution was saturated by nitrogen, sterilized by means of ultrafiltration and filled in doses of 40.5 ml into vials containing at least 600 mg of carboplatin each.
The solution was stable and no crystalline carboplatin separated out even under temperature + 5°C and 2 months storage.

### Example 6

The preparation according to Example 1 was administered to mice CBA x BL 10 in doses from 100 mg to 180 mg/kg of body weight. The same doses of fresh-made solution of lyophylized carboplatin were administered to the control groups. The relative preparation toxicity according to this invention represented 97.5% of the comparative value.

## Claims

1. Anti-tumour medical preparation on the basis of carboplatin, characterized in that the carboplatin is stabilized in sterile aqueous solution having a pH value in the range from 2.5 to 7.0 and contains 1,1-cyclobutanedicarboxylic acid and/or its sodium and/or potassium and/or ammonium salts in an amount of from 5·10⁻⁴mg/ml to 2·10⁻¹mg/ml, at least 1·10⁻⁴mg/ml of which is present in the form of mono- and/or bi-valent anion.

2. Preparation according to claim 1 characterized in that the pH value of the aqueous solution is in the range from 4.0 to 6.5.

3. Preparation according to claim 1 characterized in that the pH value of the aqueous solution is in the range from 4.5 to 6.0.

4. Preparation according to any one of the claims 1 to 3 characterized in that it contains 1,1-cyclobutanedicarboxylic acid and/or its sodium and/or potassium and/or ammonium salts in an amount of from 1·10⁻³ mg/ml to 2·10⁻¹mg/ml.

5. Preparation according to any one of the claims 1 to 4 characterized in that it contains 1,1-cyclobutanedicarboxylic acid and/or its sodium and/or potassium and/or ammonium salts in an amount of from 5·10⁻³mg/ml to 1·10⁻¹mg/ml.

6. Preparation according to any one of the claims 1 to 5 characterized in that the aqueous solution of carboplatin contains from 1·10⁻⁵ mg/ml to 6·10⁻² mg/ml ammonia or its protonized form.

7. Preparation according to any one of the claims 1 to 6 characterized in that the aqueous solution of carboplatin contains a pharmacologically acceptable ingredient from the group of saccharides.

8. Preparation according to claim 7 characterized in that the aqueous solution of carboplatin contains as a saccharide α-cyclodextrin in an amount of from 0.1 mg/ml to 10 mg/ml.

9. Preparation according to any one of the claims 1 to 8 characterized in that it contains from 0.1 mg to 25 mg of carboplatin per 1 ml solution.

10. Method for the production of the anti-tumour medical preparation according to any one of the claims 1 to 9 characterized in that the ingredients are added under aseptic conditions, successively or simultaneously dissolved in water for injection purpose, the pH value of the solution is adjusted and sterilized preferably by means of ultrafiltration, filled under aseptic conditions into containers determined for injection and/or infusion preparation while during preparation, filling into containers and storage the solution is kept under inert or oxidative atmosphere and exclusion of the short-wave light.

## Revendications

1. Préparation médicale antitumorale à base de carboplatine, caractérisée en ce que la carboplatine est stabilisée dans une solution aqueuse stérile ayant un pH compris entre 2,5 et 7,0 et en ce qu'elle contient de l'acide 1, 1-cyclobutandicarboxylique et/ou ses sels de sodium et/ou de potassium et/ou d'ammonium en quantité allant de 5 x 10⁻⁴ mg/ml à 2x10⁻¹ mg/ml, dont au moins 1x10⁻⁴ mg/ml se trouve sous forme d'anion mono- ou bivalent.

2. Préparation selon la revendication 1, caractérisée en ce que le pH de la solution aqueuse est compris entre 4,0 et 6,5.

3. Préparation selon la revendication 1, caractérisée en ce que le pH de la solution aqueuse est compris entre 4,5 et 6,0.

4. Préparation selon l'une quelconque des revendications 1 à 3, caractérisée en ce qu'elle contient de l'acide 1, 1-cyclobutandicarboxylique et/ou ses sels de sodium et/ou de potassium et/ou d'ammonium en quantité allant de 1 x 10⁻³ mg/ml à 2x10⁻¹ mg/ml.

5. Préparation selon l'une quelconque des revendications 1 à 4, caractérisée en ce qu'elle contient de l'acide 1, 1-cyclobutandicarboxylique et/ou ses sels de sodium et/ou de potassium et/ou d'ammonium en quantité allant de 5 x 10⁻³ mg/ml à 1x10⁻¹ mg/ml.

6. Préparation selon l'une quelconque des revendications 1 à 5, caractérisée en ce que la solution aqueuse de carboplatine contient de 1x10⁻⁵ mg/ml à 6x10⁻² mg/ml d'ammoniac ou sa forme protonée.

7. Préparation selon l'une quelconque des revendications 1 à 6, caractérisée en ce que la solution aqueuse de carboplatine contient un principe pharmacologiquement acceptable issu du groupe des saccharides.

8. Préparation selon la revendication 7, caractérisée en ce que la solution aqueuse de carboplatine contient à titre de saccharide de la α-cyclodextrine en quantité de 0,1 mg/ml à 10 mg/ml.

9. Préparation selon l'une quelconque des revendications 1 à 8, caractérisée en ce qu'elle contient de 0,1 mg à 25 mg de carboplatine par 1ml de solution.

10. Procédé de production de la préparation médicale antitumorale selon l'une quelconque des revendications 1 à 9, caractérisé en ce que les ingrédients sont ajoutés en milieu aseptique, dissous successivement ou simultanément dans de l'eau injectable, le pH de la solution est ajusté et celle-ci est stérilisée de préférence au moyen d'ultrafiltration, versée en milieu aseptique dans des récipients conçus pour préparations injectables et/ou à perfusion, étant précisé qu'en cours de préparation, transvasement dans des récipients et entreposage, la solution est maintenue sous atmosphère inerte ou oxydante et à l'abri de la lumière de faible longueur d'onde.

## Patentansprüche

1. Medizinisches Anti-Tumor-Mittel auf Basis von Carboplatin, dadurch gekennzeichnet, daß das Carboplatin in einer sterilen wäßrigen Lösung mit einem pH-Wert im Bereich von 2,5 bis 7,0, welche 1,1-Cyclobutandicarbonsäure und/oder deren Natrium- und/oder Kalium- und/oder Ammoniumsalze in einer Menge von 5·10⁻⁴ mg/ml bis 2·10⁻¹ mg/ml enthält, wobei wenigstens 1·10⁻⁴ mg/ml in der Form des ein- und/oder zweiwertigen Anions vorliegen, stabilisiert ist.

2. Mittel nach Anspruch 1, dadurch gekennzeichnet, daß der pH-Wert der wäßrigen Lösung im Bereich von 4,0 bis 6,5 liegt.

3. Mittel nach Anspruch 1, dadurch gekennzeichnet, daß der pH-Wert der wäßrigen Lösung im Bereich von 4,5 bis 6,0 liegt.

4. Mittel nach einem der Ansprüche 1 bis 3, dadurch gekennzeichnet, daß es 1,1-Cyclobutandicarbonsäure und/oder deren Natrium- und/oder Kalium- und/oder Ammoniumsalze in einer Menge von 1·10⁻³ mg/ml bis 2·10⁻¹ mg/ml enthält.

5. Mittel nach einem der Ansprüche 1 bis 4, dadurch gekennzeichnet, daß es 1,1-Cyclobutandicarbonsäure und/oder deren Natrium- und/oder Kalium- und/oder Ammoniumsalze in einer Menge von 5·10⁻³ mg/ml bis 1·10⁻¹ mg/ml enthält.

6. Mittel nach einem der Ansprüche 1 bis 5, dadurch gekennzeichnet, daß die wäßrige Lösung von Carboplatin von 1·10⁻⁵ mg/ml bis 6·10⁻² mg/ml Ammoniak oder dessen protonierte Form enthält.

7. Mittel nach einem der Ansprüche 1 bis 6, dadurch gekennzeichnet, daß die wäßrige Lösung von Carboplatin einen pharmazeutisch verträglichen Bestandteil aus der Gruppe der Saccharide enthält.

8. Mittel nach Anspruch 7, dadurch gekennzeichnet, daß die wäßrige Lösung von Carboplatin als Saccharid α-Cyclodextrin in einer Menge von 0,1 mg/ml bis 10 mg/ml enthält.

9. Mittel nach einem der Ansprüche 1 bis 8, dadurch gekennzeichnet, daß es pro 1 ml Lösung von 0,1 mg bis 25 mg Carboplatin enthält.

10. Verfahren zur Herstellung des medizinischen Anti-Tumor-Mittels nach einem der Ansprüche 1 bis 9, dadurch gekennzeichnet, daß die Bestandteile unter aseptischen Bedingungen nacheinander oder gleichzeitig in Wasser für Injektionszwecke gelöst werden, der pH-Wert der Lösung eingestellt wird und die Lösung vorzugsweise durch Ultrafiltration sterilisiert wird, die Lösung unter aseptischen Bedingungen in für die Injektion und/oder Infusion des Mittels bestimmte Behältnisse abgefüllt wird, wobei die Lösung während der Herstellung, des Abfüllens in die Behältnisse und der Lagerung unter einer inerten bzw. oxidativen Atmosphäre unter Ausschluß von kurzwelligem Licht aufbewahrt wird.
